# EUROPÄISCHE PATENTSCHRIFT

(11) **EP 1 812 117 B1**
(45) Veröffentlichungstag und Bekanntmachung des Hinweises auf die Patenterteilung: **02.01.2008**
(21) Anmeldenummer: 05732176.2
(22) Anmeldetag: 08.04.2005
(51) Int. Cl.: A61Q 5/00, A61K 8/44, A61K 8/362

(54) **VERFAHREN ZUR RESTRUKTURIERUNG KERATINISCHER FASERN**
METHOD FOR RESTRUCTURING KERATIN FIBERS
PROCEDE DE RESTRUCTURATION DE FIBRES KERATINIQUES

(30) Priorität: 18.05.2004 DE 102004024506
(43) Veröffentlichungstag der Anmeldung: 01.08.2007
(73) Patentinhaber: Henkel Kommanditgesellschaft auf Aktien, 40589 Düsseldorf (DE)
(72) Erfinder: KAINZ, Sabine, 47447 Moers (DE); HUCHEL, Ursula, 50670 Köln (DE); KALISCHKE, Swenja, 47509 Rheurdt (DE)
(86) Internationale Anmeldenummer: PCT/EP2005/003696
(87) Internationale Veröffentlichungsnummer: WO 2005/115314

(56) Entgegenhaltungen:
- EP-A- 1 226 814
- DE-A1- 10 051 774
- US-A- 4 412 943

## Beschreibung

Die Erfindung betrifft ein Verfahren zur Restrukturierung keratinischer Fasern, bei dem eine Keratinfaser mit Cystin und mit mindestens einer Dicarbonsäure mit 2 bis 10 Kohlenstoffatomen in Kontakt gebracht wird. Ferner betrifft die Erfindung Zubereitungen zur Anwendung in diesen Verfahren.

Keratinischen Fasern, insbesondere Haaren kommen als festem Bestandteil des menschlichen Körpers und als wesentlichem Bestandteil von menschlicher Kleidung und Heimtextilen, eine wichtige Bedeutung im Alltagsgeschehen zu. Die Behandlung mit Wasch-, Reinigungs-, Styling- und Färbeprodukten, zu Reinigungs- und Gestaltungszwecken, sowie deren Exposition gegenüber Umwelteinflüssen, wie Ozon, Salz- und Chlorwasser, IR-, UV- und Wärmestrahlung (Fönen) führen im Laufe der Zeit zu einer kumulativen Schädigung der Fasern und somit zu einer Verminderung ihrer Qualität. Beispielsweise sind sowohl die Reinigung von Haaren mit Shampoos als auch die dekorative Gestaltung der Frisur durch Färben oder Dauerwellen Eingriffe, die die natürliche Struktur und die Eigenschaften der Haare beeinflussen. Folglich können nach einer solchen Behandlung beispielsweise die Nass- und Trockenkämmbarkeit, Halt, Fülle, Glanz und Taktilität des Haares unbefriedigend sein. Im Fall gefärbter Haare kann insbesondere bei häufiger Haarwäsche weiterhin der Halt der Farbe auf dem Haar unbefriedigend sein, so daß es zu einem allmählichen Ausbluten der Farbe kommt.

Nicht zuletzt durch die starke Beanspruchung der Haare, beispielsweise durch das Färben oder Dauerwellen wie auch durch die Reinigung der Haare mit Shampoos und durch Umweltbelastungen, nimmt die Bedeutung von Pflegeprodukten mit ausreichend starker und möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares verbessert sein oder die Haare vor einer erhöhten Splißrate geschützt sein.

Es ist daher seit langem üblich, die Haare einer speziellen Nachbehandlung zu unterziehen. Dabei werden, meist in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.

Pflegende Zusätze und Filmbildner werden häufig auch Dauerwellmitteln zugesetzt, ohne aber dabei die Haarstruktur deutlich zu verbessern. Dazu werden beispielsweise hochmolekulare Polymere eingesetzt, die auf die oberste Schicht von Haut und Haaren aufziehen und dort einen äußerlichen, subjektiv wahrnehmbar verbesserten Griff des Haares erzeugen. Die Strukturschädigung im Inneren des Haares, die bei Dauerwellen vor allem durch den Reduktionsprozess verursacht wird, kann dadurch aber nicht vermindert werden, da die Substanzen aufgrund ihrer Größe nicht in das Haar eindringen können. Außerdem ist die Dauerhaftigkeit der Effekte der strukturverbessernden Zusätze häufig unbefriedigend, da diese dem Haar nur oberflächlich anhaften.

Es hat Versuche gegeben, diesem Problem abzuhelfen, indem monomere Verbindungen direkt auf dem Haar polymerisiert wurden. So werden nach der Lehre der US 5 362 486 bestimmte Urethan-Oligomere mit endständigen Bisulfit- oder Acrylatgruppen auf das Haar aufgebracht und dann polymerisiert, wobei auf dem Haar in situ anhaftende Polymere gebildet werden. Durch dieses Verfahren werden die Oberflächeneigenschaften von Haaren in günstiger Weise beeinflußt, wie z.B. Volumen, Glanz, Halt, Kämmbarkeit, sowie Resistenz gegen die Aufnahme von Feuchtigkeit und Luftverunreinigungen und gegen den Verlust von Haarfarben. Bei dem Verfahren findet auf dem Haar eine radikalische Polymerisation statt, d.h. das Haar muß mit Radikalbildnern wie z.B. Benzoylperoxid behandelt werden.

Aus der EP-A 1174112 sind Haarbehandlungsmittel bekannt, die neben einer organischen Säure als weitere zwingende Bestandteile ein organisches Lösungsmittel, ein kationisches Tensid und einen höheren Alkohol enthalten und zur Reparatur von Poren in Haaren dienen.

Die Aufgabe der Erfindung bestand darin, ein Verfahren zur Restrukturierung von Keratinfasern bereitzustellen, welches Vorteile gegenüber dem Stand der Technik aufweist und eine ausreichende Wirksamkeit und Wirkdauer ermöglicht. Das Verfahren sollte nicht nur unter faserschonenden Bedingungen durchführbar sein, sondern auch physiologisch unbedenklich sein, d.h. insbesondere ohne die Verwendung toxikologisch bedenklicher Stoffe wie Radikalbildnern auskommen.

Die erfindungsgemäße Aufgabe wird gelöst durch ein Verfahren, bei dem keratinische Fasern mit Cystin und mit mindestens einer Dicarbonsäure mit 2 bis 10 Kohlenstoffatomen in Kontakt gebracht wird.

Überraschenderweise wurde festgestellt, dass mit Hilfe einer Kombination aus Cystin und mindestens einer Dicarbonsäure mit 2 bis 10 Kohlenstoffatomen die Zug-Dehnungseigenschaften keratinischer Fasern verbessert und ihre Festigkeit erhöht werden kann, ohne daß es zu einer Schädigung der Faser kommt.

Die DE-A 10051774 beschreibt die Verwendung kurzkettiger Carbonsäuren mit einem Molekulargewicht kleiner als 750 als Wirkstoff zur Restrukturierung keratinischer Fasern in kosmetischen Mitteln. Die gleichzeitige Verwendung von Cystin wird dort weder beschrieben noch nahegelegt, ebensowenig wie etwaige besondere Effekte einer solchen Kombination.

Ein erster Gegenstand der vorliegenden Erfindung ist daher ein Verfahren zur Restrukturierung keratinischer Fasern, bei dem eine Keratinfaser mit Cystin und mit mindestens einer Dicarbonsäure mit 2 bis 10 Kohlenstoffatomen in Kontakt gebracht wird

Unter keratinischen Fasern sind erfindungsgemäß Pelze, Wolle, Federn, Seide und Haare, insbesondere aber menschliche Haare zu verstehen.

Im Rahmen der vorliegenden Erfindung wurde gefunden, daß durch den Einsatz des erfindungsgemäßen Verfahrens die innere und äußere Struktur von keratinischen Fasern verändert werden kann, d.h. mit anderen Worten, daß eine Restrukturierung keratinischer Fasern ermöglicht wird. Unter Restrukturierung wird im Sinne der vorliegenden Erfindung insbesondere eine Faserverstärkung, eine Reißkrafterhöhung und/oder eine Verringerung der durch verschiedenartigste Einflüsse entstandenen Schädigungen keratinischer Fasern verstanden. Hierbei spielt beispielsweise die Wiederherstellung der natürlichen Festigkeit eine wesentliche Rolle. Restrukturierte Fasern können sich beispielsweise durch eine erhöhte Reißkraft, eine erhöhte Festigkeit, eine erhöhte Elastizität und/oder ein erhöhtes Volumen auszeichnen, welches sich beispielsweise bei einer Frisur in einer größeren Fülle zeigen kann. Weiterhin können sie einen verbesserten Glanz, einen verbesserten Griff und/oder eine leichtere Kämmbarkeit aufweisen.

Das erfindungsgemäße Verfahren dient der Stärkung, dem Schutz und der Reparatur keratinischer Fasern und ist ganz besonders zur Verbesserung der Haarstruktur und/oder der Verstärkung von menschlichen Haaren geeignet. Insbesondere werden Fasereigenschaften wie Festigkeit, Elastizität oder Volumen im Sinne einer Zunahme dieser Eigenschaften positiv beeinflusst. Außerdem eignet sich das Verfahren zu Stylingzwecken, wie Formgebung und Formerhalt.
Das erfindungsgemäße Verfahren ist weiterhin geeignet, Fasern vor dem schädigenden Einfluß von Licht zu schützen.

Das erfindungsgemäße Verfahren erfordert keine toxikologisch bedenklichen Stoffe, wie z.B. Radikalbildner bzw. intermediär auftretende Radikale.

Im Sinne der Erfindung ist unter einer Dicarbonsäure eine Verbindung mit mindestens zwei Carboxylgruppen zu verstehen, d.h. z.B. auch eine Verbindung mit drei oder mehr Carboxylgruppen.

In einer bevorzugten Ausführungsform der Erfindung enthält die in dem erfindungsgemäßen Verfahren verwendete Dicarbonsäure jedoch zwei und nicht mehr, d.h. genau zwei Carboxylgruppen.

In bevorzugten Ausführungsformen der Erfindung werden Dicarbonsäuren eingesetzt, die ausgewählt sind aus der Gruppe, die gebildet wird von Dicarbonsäuren HOOC-(CH₂)ₙ-COOH mit n=0 bis 8, Maleinsäure, Fumarsäure, Sorbinsäure sowie Gemischen dieser Stoffe. Zu den Dicarbonsäuren HOOC-(CH₂)ₙ-COOH mit n=1 bis 8 zählen beispielsweise Oxalsäure, Malonsäure, Bernsteinsäure, Glutarsäure, Adipinsäure, Pimelinsäure und Azelainsäure.

In einer besonders bevorzugten Ausführungsformen der Erfindung wird als Dicarbonsäure Bernsteinsäure eingesetzt.

In dem erfindungsgemäßen Verfahren wird Cystin und die mindestens eine Dicarbonsäure in einem Gewichtsverhältnis von 99 zu 1 bis 1 zu 99, vorzugsweise von 10 zu 1 bis 1 zu 10, und insbesondere von 2 zu 1 bis 1 zu 2 eingesetzt.

Ein weiterer Gegenstand der Erfindung ist die Verwendung einer Kombination aus Cystin und mindestens einer Dicarbonsäure mit 2 bis 10 Kohlenstoffatomen zur Restrukturierung von keratinischen Fasern, insbesondere von Haaren, wobei die Restrukturierung insbesondere eine Faserverstärkung umfaßt.

Weiter betrifft die vorliegende Erfindung eine Faser, insbesondere eine keratinische Faser, die erhältlich ist durch das zuvor beschriebene Verfahren.

Die Temperatur beim In-Kontakt-Bringen der Dicarbonsäure mit der Faser liegt vorzugsweise in einem Bereich von etwa 15 bis etwa 40°C.

Das In-Kontakt-Bringen der Faser mit Cystin und der Dicarbonsäure kann so erfolgen, daß daneben weitere Stoffe zugegen sind. Diese Stoffe werden vorzugsweise so ausgewählt, daß sie einen zur Behandlung der Faser geeigneten Träger für das Cystin und/oder die Dicarbonsäure bilden.

Das In-Kontakt-Bringen kann so erfolgen, dass die Faser nacheinander mit mindestens zwei Zubereitungen in Kontakt gebracht wird, von denen eine Cystin enthält und eine weitere mindestens eine dicarbonsäure enthält.

Vorzugsweise wird das erfindungsgemäße Verfahren so ausgeführt, daß eine Faser mit einer Zubereitung in Kontakt gebracht wird, die sowohl Cystin als auch mindestens eine Dicarbonsäure enthält.

Für den Fall, daß die Faser ein Haar ist, bilden die neben Cystin und/oder der Dicarbonsäure in der Zubereitung vorliegenden Stoffe vorzugsweise eine Zusammensetzung von der Art, wie sie dem Haarkosmetik-Fachmann als "Wellmittel" geläufig ist.

Die Träger für die in dem erfindungsgemäßen Verfahren verwendeten Zubereitungen können fest, flüssig, gelförmig oder pastös sein. Sie sind vorzugsweise ausgewählt aus wässrigen Systemen, natürlichen oder synthetischen Ölen, Wasser in-Öl- oder Öl-in-Wasser-Emulsionen. Derartige Systeme und Verfahren zu deren Herstellung sind im Stand der Technik bekannt, auf den hiermit verwiesen wird. Die Zubereitungen können als Creme, Gel oder Flüssigkeit formuliert sein. Weiterhin ist es möglich, die Mittel in Form von Schaumaerosolen zu konfektionieren, die mit einem verflüssigten Gas wie z. B. Propan-Butan-Gemischen, Stickstoff, CO₂, Luft, NO₂, Dimethylether, Fluorchlorkohlenwasserstofftreibmitteln oder Gemischen davon in Aerosolbehältern mit Schaumventil abgefüllt werden. Bevorzugt werden die einzelnen Komponenten des erfindungsgemäßen Verfahrens als Creme, Gel oder Flüssigkeit angewendet. Weiterhin können die erfindungsgemäß verwendeten Zubereitungen zwei- oder mehrphasig vorliegen. Zwei- und Mehrphasensysteme sind Systeme, bei denen mindestens zwei separate, kontinuierliche Phasen vorliegen. Beispielsweise können in solchen Systemen eine wässrige Phase und eine oder mehrere, z.B. zwei, nicht miteinander mischbare, nichtwässrige Phasen, separat voneinander vorliegen. Ebenso möglich sind beispielsweise eine Wasser-in-Öl-Emulsion und eine davon separiert vorliegende wässrige Phase bzw. eine Wasser-in-Öl-Emulsion und eine davon separiert vorliegende wässrige Phase.

Gegenstand der Erfindung ist weiterhin eine Zubereitung zur Verwendung in dem erfindungsgemäßen Verfahren. Die Zubereitung ist vorzugsweise wäßrig und enthält
(a) 0,01 bis 20 Gew.-% Cystin und
(b) 0,01 bis 20 Gew.-% mindestens einer Dicarbonsäure mit 2 bis 10 Kohlenstoffatomen, wie im vorangehenden Text definiert,
jeweils bezogen auf das Gesamtgewicht der Zubereitung.

Als weitere Bestandteile der in dem erfindungsgemäßen Verfahren verwendeten Zubereitungen können die im folgenden beschriebenen Wirksubstanzen vorteilhaft eingesetzt werden.

Als Wirksubstanzen geeignet sind Tenside. Unter dem Begriff Tenside werden grenzflächenaktive Substanzen, die an Ober- und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wäßriger Lösung stark hydratisiert sind. Weitergehende Definitionen und Eigenschaften von Tensiden finden sich in "H.-D.Dörfler, Grenzflächen- und Kolloidchemie, VCH Verlagsgesellschaft mbH. Weinheim, 1994". Die zuvor wiedergegebene Begriffsbestimmung findet sich ab S. 190 in dieser Druckschrift.

Als anionische Tenside eignen sich in erfindungsgemäßen Zubereitungen prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II),

   R¹(OCH₂CH₂)ₙ-O-P(O)(OX)(OR²) (II)

   in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (III)

   R⁷CO(AlkO)ₙSO₃M (III)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (IV) 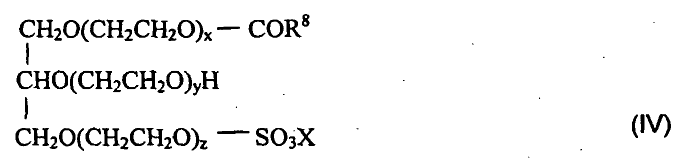 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, wie sie beispielsweise in der EP-B1 0 561 825, der EP-B1 0 561 999, der DE-A1 42 04 700 oder von A.K.Biswas et al. in J. Am. Oil. Chem. Soc. 37, 171 (1960) und F.U.Ahmed in J. Am. Oil. Chem. Soc. 67, 8 (1990) beschrieben worden sind,
- Amidethercarbonsäuren wie sie in der EP 0 690 044 beschrieben sind,
- Kondensationsprodukte aus C₈ - C₃₀ - Fettalkoholen mit Proteinhydrolysaten und/oder Aminosäuren und deren Derivaten,welche dem Fachmann als Eiweissfettsäurekondensate bekannt sind, wie beispielsweise die Lamepon^{®} - Typen, Gluadin^{®}- Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten. Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (V)

   R¹CO-(OCH₂CHR²)_{w}OR³ (V)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (VI),

   R⁴O-[G]ₚ (VI)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Joumal Heft 8, 598 (1995) verwiesen.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (VII), 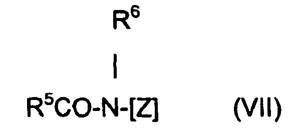 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H.Kelkenberg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (VIII) wiedergegeben werden: 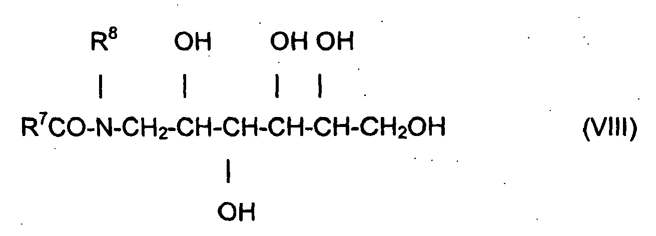
   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-1V), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Zubereitungen bevorzugt in Mengen von 0,1 - 20 Gew.-%, bezogen auf die gesamte Zubereitung, enthalten sein. Mengen von 0,5 - 15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Zubereitung kann in einer weiteren Ausführungsform der Erfindung einen Komplexbildner enthalten, beispielsweise EDTA, NTA, β-Alanindiessigsäure, eine Phosphonsäure oder Gemische aus diesen Substanzen.

Als weitere Wirksubstanzen eignen sich Polyole wie beispielsweise Glycerin und Partialglycerinether, 2-Ethyl-1,3-hexandiol, 1,3-Butandiol, 1,4-Butandiol, 1,2-Propandiol, 1,3-Propandiol, Pentandiole, beispielsweise 1,2-Pentandiol, Hexandiole, beispielsweise 1,2-Hexandiol oder 1,6-Hexandiol, Dodekandiol, insbesondere 1,2-Dodekandiol, Neopentylglykol und Ethylenglykol. Insbesondere 2-Ethyl-1,3-hexandiol, 1,2-Propandiol, 1,3-Propandiol und 1,3-Butandiol haben sich als besonders gut geeignet erwiesen.

Diese Polyole sind in den erfindungsgemäß verwendeten Zubereitungen bevorzugt in Mengen von 1 - 10, insbesondere 2 - 10, Gew.-%, bezogen auf die gesamte Zubereitung, enthalten.

Erfindungsgemäß können selbstverständlich auch mit Wasser nur begrenzt mischbare Alkohole eingesetzt werden, insbesondere, wenn Mehrphasensysteme erhalten werden sollen.

Unter "mit Wasser begrenzt mischbar" werden solche Alkohole verstanden, die in Wasser bei 20 °C zu nicht mehr als 10 Gew.-%, bezogen auf die Wassermasse, löslich sind.

Als weitere Wirksubstanzen können Fettstoffe eingesetzt werden. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten.

Als Fettsäuren können eingesetzt werden lineare und/oder verzweigte, gesättigte und/oder ungesättigte Fettsäuren mit 6 - 30 Kohlenstoffatomen in Mengen von 0,1 - 15 Gew.%, bezogen auf das gesamte Mittel. Als Fettalkohole können eingesetzt werden gesättigte, ein- oder mehrfach ungesättigte, verzweigte oder unverzweigte Fettalkohole mit C₆- C₃₀- Kohlenstoffatomen in Mengen von 0,1 - 30 Gew.-%, bezogen auf die gesamte Zubereitung.

Natürliche und synthetische kosmetische Ölkörper, welche erfindungsgemäß als Wirkstoff verwendet werden können, sind insbesondere:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyln-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyt)-cyclohexan (Cetiol^{®} S) und Di-n-octylether (Cetiol^{®} OE) können bevorzugt sein.

Die Einsatzmenge der natürlichen und synthetischen kosmetischen Ölkörper in den erfindungsgemäß verwendeten Zubereitungen beträgt üblicherweise 0,1 - 30 Gew.%, bezogen auf die gesamte Zubereitung, bevorzugt 0,1 - 20 Gew.-%, und insbesondere 0,1-15 Gew.-%.

Die Gesamtmenge an Öl- und Fettkomponenten in den erfindungsgemäßen Zubereitungen beträgt üblicherweise 0,1.- 75 Gew.-%, bezogen auf die gesamte Zubereitung. Mengen von 0,1 - 35 Gew.-% sind erfindungsgemäß bevorzugt.

Weiterhin hat es sich gezeigt, daß vorteilhafterweise Polymere im Rahmen des erfindungsgemäßen Verfahrens eingesetzt werden. In einer bevorzugten Ausführungsform werden den erfindungsgemäß verwendeten Zubereitungen daher Polymere zugesetzt, wobei sich sowohl kationische, anionische, amphotere als auch nichtionische Polymere als wirksam erwiesen haben.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert der Zubereitung eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (IX), 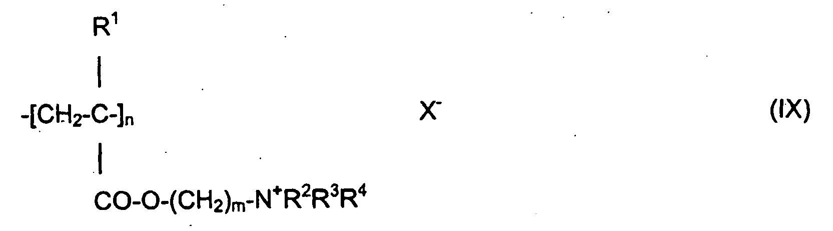 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (II) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vemetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Saicare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (II) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Coming; ein stabilisiertes Trimethylsilylamodimethicon), Dow Corning^{®} 929 Emulsion (enthaltend ein hydroxyl-amino-modifiziertes. Silicon, das auch als Amodimethicone bezeichnet wird), SM-2059 (Hersteller: General Electric), SLM-55067 (Hersteller: Wacker) sowie Abit^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quatemierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquatemium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäß einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Bei den anionischen Polymeren, welche in den Zubereitungen des erfindungsgemäßen Verfahrens verwendet werden können, handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Beispielsweise ist ein solches Homopolymer der 2-Acrylamido-2-methylpropansulfon-säure, unter der Bezeichnung Rheothik^{®}11-80 im Handel erhältlich.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere - mit Sulfonsäuregruppen-haltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls gut geeignete Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere in allen wäßrigen Zubereitungen des erfindungsgemäßen Verfahrens amphotere Polymere verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO- oder -SO₃⁻- Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrytamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel (X),

   R¹-CH=CR²-CO-Z-(CₙH₂ₙ)-N⁽⁺⁾R³R⁴R⁵ A⁽⁻⁾ (X)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (XI),

   R⁶-CH=CR⁷-COOH (XI)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (a). Als Monomeres (b) für die genannten Polymerisate wird bevorzugt Acrylsäure verwendet.

Weiterhin können in allen wäßrigen Zubereitungen des erfindungsgemäßen Verfahrens nichtionogene Polymere enthalten sein. Es kann bevorzugt sein, diese in der Zubereitung (O) zu verwenden.

Geeignete nichtionogene Polymere sind beispielsweise:
- VinylpyrrolidonNinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone gemäß der EP 0612759 B1.

Es ist erfindungsgemäß auch möglich, daß die verwendeten Zubereitungen mehrere, insbesondere zwei verschiedene Polymere gleicher Ladung und/oder jeweils ein ionisches und ein amphoteres und/oder nicht ionisches Polymer enthalten.

Die Polymere sind in den erfindungsgemäß verwendeten Zubereitungen bevorzugt in Mengen von 0,05 bis 10 Gew.-%, bezogen auf die gesamte Zubereitung, enthalten. Mengen von 0,1 bis 5, insbesondere von 0,1 bis 3 Gew.-%, sind besonders bevorzugt.

Weiterhin können in den erfindungsgemäß verwendeten Zubereitungen Proteinhydrolysate und/oder neben Cystin weitere Aminosäuren und deren Derivate enthalten sein. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Die Proteinhydrolysate oder deren Derivate sind in den erfindungsgemäß verwendeten Zubereitungen bevorzugt in Mengen von 0,1 bis 10 Gew.-%, bezogen auf die gesamte Zubereitung, enthalten. Mengen von 0,1 bis 5 Gew.-% sind besonders bevorzugt.

Weiterhin können 2-Pyrrolidinon-5-carbonsäure und/oder deren Derivate in den Zubereitungen des erfindungsgemäßen Verfahrens verwendet werden. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Zubereitungen betragen 0,05 bis 10 Gew.%, bezogen auf die gesamte Zubereitung, besonders bevorzugt 0,1 bis 5, und insbesondere 0,1 bis 3 Gew.%.

Ebenfalls als vorteilhaft hat sich die Verwendung von Vitaminen, Provitaminen und Vitaminvorstufen sowie deren Derivaten erwiesen.

Dabei sind erfindungsgemäß solche Vitamine, Pro-Vitamine und Vitaminvorstufen bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäß verwendeten Zubereitungen enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Zubereitungen bevorzugt in Mengen von 0,05 bis 1 Gew.%, bezogen auf die gesamte Zubereitung, enthalten ist.
   Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäß verwendeten Zubereitungen bevorzugt in Mengen von 0,05 - 10 Gew.-%, bezogen auf die gesamte Zubereitung, enthalten. Mengen von 0,1-5 Gew.-% sind besonders bevorzugt.
- Vitamin B₆ (Pyridoxin sowie Pyridoxamin und Pyridoxal),
- Vitamin C (Ascorbinsäure). Vitamin C wird in den erfindungsgemäß verwendeten Zubereitungen bevorzugt in Mengen von 0,1 bis 3 Gew.-%, bezogen auf die gesamte Zubereitung eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.
- Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäß verwendeten Zubereitungen bevorzugt in Mengen von 0,05-1 Gew.-%, bezogen auf die gesamte Zubereitung, enthalten.
- Vitamin F. Unter dem Begriff "Vitamin F" werden üblicherweise essentielle Fettsäuren, insbesondere Linolsäure, Linolensäure und Arachidonsäure, verstanden.
- Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäß verwendeten Zubereitungen bevorzugt in Mengen von 0,0001 bis 1,0 Gew.-%, insbesondere in Mengen von 0,001 bis 0,01 Gew.-% enthalten.

Bevorzugt enthalten die erfindungsgemäß verwendeten Zubereitungen Vitamine, Provitamine und Vitaminvorstufen aus den Gruppen A, B, E und H.

Panthenol, Pantolacton, Pyridoxin und seine Derivate sowie Nicotinsäureamid und Biotin sind besonders bevorzugt.

Schließlich können in den Zubereitungen des erfindungsgemäßen Verfahrens Pflanzenextrakten verwendet werden.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel.

Ganz besonders für die erfindungsgemäße Verwendung geeignet sind die Extrakte aus Grünem Tee, Mandel, Aloe Vera, Kokosnuß, Mango, Aprikose, Limone, Weizen, Kiwi und Melone.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Zubereitungen Mischungen aus mehreren, insbesondere aus zwei, verschiedenen Pflanzenextrakten einzusetzen.

Weiterhin ist es erfindungsgemäß bevorzugt, Hydroxycarbonsäuren und hierbei wiederum insbesondere die Dihydroxy-, Trihydroxy- und Polyhydroxycarbonsäuren sowie die Dihydroxy-, Trihydroxy- und Polyhydroxy- di-, tri- und polycarbonsäuren einzusetzen. Hierbei hat sich gezeigt, daß neben den Hydroxycarbonsäuren auch die Hydroxycarbonsäureester sowie die Mischungen aus Hydroxycarbonsäuren und deren Estern als auch polymere Hydroxycarbonsäuren und deren Ester ganz besonders bevorzugt sein können. Bevorzugte Hydroxycarbonsäureester sind beispielsweise Vollester der Glycolsäure, Milchsäure, Äpfelsäure, Weinsäure oder Citronensäure. Weitere grundsätzlich geeigneten Hydroxycarbonsäureester sind Ester der β-Hydroxypropionsäure, der Tartronsäure, der D-Gluconsäure, der Zuckersäure, der Schleimsäure oder der Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C12-C15-Fettalkoholen besonders bevorzugt. Ester dieses Typs sind im Handel erhältlich, z.B. unter dem Warenzeichen Cosmacol^{®} der EniChem, Augusta Industriale. Besonders bevorzugte Polyhydroxypolycarbonsäuren sind Polymilchsäure und Polyweinsäure sowie deren Ester.

In einer weiteren bevorzugten Ausführungsform werden in den Zubereitungen des erfindungsgemäßen Verfahrens Emulgatoren verwendet. Emulgatoren bewirken an der Phasengrenzfläche die Ausbildung von wasser- bzw. ölstabilen Adsorptionsschichten, welche die dispergierten Tröpfchen gegen. Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe.
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Zubereitungen enthalten die Emulgatoren bevorzugt in Mengen von 0,1 - 25 Gew.-%, insbesondere 0,1 - 3 Gew.-%, bezogen auf die gesamte Zubereitung.

Bevorzugt können die erfindungsgemäßen Zubereitungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Als weitere Wirksubstanzen können heterocyclische Verbindungen wie beispielsweise Derivate von Imidazol, Pyrrolidin, Piperidin, Dioxolan, Dioxan, Morpholin und Piperazin eingesetzt werden. Weiterhin eignen sich Derivate dieser Verbindungen wie beispielsweise die C₁₋₄-Alkyl-Derivate, C₁₋₄-Hydroxyalkyl-Derivate und C₁₋₄-Aminoalkyl-Derivate. Bevorzugte Substituenten, die sowohl an Kohlenstoffatomen als auch an Stickstoffatomen der heterocyclischen Ringsysteme positioniert sein können, sind Methyl-, Ethyl-, β-Hydroxyethyl- und β-Aminoethyl-Gruppen. Bevorzugt enthalten diese Derivate 1 oder 2 dieser Substituenten.

Erfindungsgemäß bevorzugte Derivate heterocyclischer Verbindungen sind beispielsweise 1-Methylimidazol, 2-Methylimidazol, 4(5)-Methylimidazol, 1,2-Dimethylimidazol, 2-Ethylimidazol, 2-Isopropyümidazol, N-Methylpyrrolidon, 1-Methylpiperidin, 4-Methylpiperidin, 2-Ethylpiperidin, 4-Methylmorpholin, 4-(2-Hydroxyethyl)morpholin, 1-Ethylpiperazin, 1-(2-Hydroxyethyl)piperazin, 1-(2-Aminoethyl)piperazin. Weiterhin erfindungsgemäß bevorzugte Imidazolderivate sind Biotin, Hydantoin und Benzimidazol.

Unter diesen heterocyclischen Wirksubstanzen sind die Mono- und Dialkylimidazole, Biotin und Hydantoin besonders bevorzugt.

Diese heterocyclischen Verbindungen sind in den erfindungsgemäßen Zubereitungen in Mengen von 0,5 bis 10 Gewichts-%, bezogen auf die gesamte Zubereitung, enthalten. Mengen von 2 bis 6 Gew.-% haben sich als besonders geeignet erwiesen.

Weitere Wirksubstanzen, welche in allen erfindungsgemäß verwendeten wäßrigen Zubereitungen enthalten sein können, sind erfindungsgemäß Aminosäuren und Aminosäurederivate. Aus der Gruppe der Aminosäuren haben sich insbesondere Arginin, Citrullin, Histidin, Ornithin und Lysin als erfindungsgemäß geeignet erwiesen. Die Aminosäuren können sowohl als freie Aminosäure, als auch als Salze, z. B. als Hydrochloride eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen.

Erfindungsgemäß besonders bevorzugt sind Arginin sowie dessen Salze und argininreiche Oligopeptide.

Diese Aminosäuren bzw. Derivate sind in den erfindungsgemäßen Zubereitungen in Mengen von 0,5 bis 10 Gewichts-%, bezogen auf die gesamte Zubereitung, enthalten. Mengen von 2 bis 6 Gew.-% haben sich als besonders geeignet erwiesen.

Zusätzlich kann es sich als vorteilhaft erweisen, wenn in den erfindungsgemäßen Zubereitungen Penetrationshilfsstoffe und/ oder Quellmittel enthalten sind. Hierzu sind beispielsweise zu zählen Harnstoff und Harnstoffderivate, Guanidin und dessen Derivate, Arginin und dessen Derivate, Wasserglas, Imidazol und dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol. Die Penetrationshilfsstoffe und Quellmittel sind in den erfindungsgemäß eingesetzten Zubereitungen in Mengen von 0,1 bis 20 Gewichts-%, bezogen auf die gesamte Zubereitung, enthalten. Mengen von 01, bis 10 Gewichts-% sind bevorzugt.

Weiterhin können die erfindungsgemäßen Zubereitungen, insbesondere wenn es sich um Wellotionen handelt, wellkraftverstärkende Komponenten, insbesondere Harnstoff, Imidazol und die oben genannten Diole enthalten. Bezüglich näherer Informationen zu solchen wellkraftverstärkenden Komponenten wird auf die Druckschriften DE-OS 44 36 065 und EP-B1-363 057 verwiesen, auf deren Inhalt ausdrücklich Bezug genommen wird.

Die wellkraftverstärkenden Verbindungen können in den erfindungsgemäßen Zubereitungen in Mengen von 0,5 bis 5 Gew.%, bezogen auf die gesamte Zubereitung, enthalten sein. Mengen von 1 bis 4 Gew.-% haben sich als ausreichend erwiesen, weshalb diese Mengen besonders bevorzugt sind.

In erfindungsgemäßen zubereitungen, welche ein Fixiermittel darstellen, wird neben mindestens einem Oxidationsmittel, wie z.B. Wasserstoffperoxid, vorzugsweise weiterhin ein zur Stabilisierung wäßriger Wasserstoffperoxidzubereitungen üblicher Stabilisator eingesetzt. Der pH-Wert solcher wäßriger H₂O₂-Zubereitungen, die üblicherweise etwa 0,5 bis 15 Gew.-%, gebrauchsfertig in der Regel etwa 0,5 - 3 Gew.-%, H₂O₂ enthalten, liegt bevorzugt bei 2 bis 6, insbesondere 2 bis 4; er wird durch Säuren, bevorzugt Phosphorsäure, Phosphonsäuren und/oder Dipicolinsäure, eingestellt. Fixiermittel auf Bromat-Basis enthalten die Bromate üblicherweise in Konzentrationen von 1 bis 10 Gew.-% eingesetzt und der pH-Wert der Lösungen wird auf 4 bis 7 eingestellt. Erfindungsgemäß besonders bevorzugt kann die Verwendung von Fixiermittel-Konzentraten sein, die vor Anwendung mit Wasser verdünnt werden.

Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel, oder auch Enzyme verwendet werden, die Elektronen aus geeigneten Entwickler-komponenten (Reduktionsmittel) auf Luftsauerstoff übertragen. Bevorzugt sind dabei Oxidasen wie Tyrosinase, Ascorbatoxidase und Laccase aber auch Glucoseoxidase, Uricase oder Pyruvatoxidase. Weiterhin sei das Vorgehen genannt, die Wirkung geringer Mengen (z. B. 1% und weniger, bezogen auf das gesamte Mittel) Wasserstoffperoxid durch Peroxidasen zu verstärken.

Die erfindungsgemäßen Fixiermittel können auch als Feststoffe formuliert werden. Sie enthalten das Oxidationsmittel dann in Form eines Festkörpers, z.B. Kalium- oder Natriumbromat. Ebenfalls möglich und bevorzugt ist, das Oxidationsmittel als 2-Komponenten-System zu formulieren. Die beiden Komponenten, von denen die eine bevorzugt eine Wasserstoffperoxidlösung oder eine wäßrige Lösung eines anderen Oxidationsmittels ist und die andere die übrigen Bestandteile, insbesondere pflegende Substanzen und/oder Reduktionsmittel, enthält, werden ebenfalls erst kurz vor der Anwendung vermischt.

Neben den bereits genannten Komponenten können die in Schritt (b) des erfindungsgemäßen Verfahrens verwendeten Zubereitungen als oberflächenaktive Verbindungen kationische Tenside vom Typ der quarternären Ammoniumverbindungen, der Esterquats und der Amidoamine enthalten. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quatemierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäß verwendeten Zubereitungen bevorzugt in Mengen von 0,05 bis 10 Gew.-%, bezogen auf die gesamte Zubereitung, enthalten. Mengen von 0,1 bis 5 Gew.-% sind besonders bevorzugt. Weiterhin sind als konditionierende Wirkstoffe geeignet Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200 und DC 1401 vertriebenen Produkte sowie das Handelsprodukt Fancorsil^{®} LIM-1.

Erfindungsgemäß als konditionierende Wirkstoffe besonders bevorzugt sind kationische Silikonöle wie beispielsweise die im Handel erhältlichen Produkte Q2-7224 (Hersteller: Dow Corning; ein stabilisiertes Trimethylsilylamodimethicon), Dow Corning^{®} 939 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80). Ein geeignetes anionisches Silikonöl ist das Produkt Dow Corning^{®}1784.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline, sowie Silikonöle,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- konditionierende Wirkstoffe wie Paraffinöle, pflanzliche Öle, z. B. Sonnenblumenöl, Orangenöl, Mandelöl, Weizenkeimöl und Pfirsichkernöl sowie
- quaternierte Amine wie Methyl-1-alkylamidoethyl-2-alkylimidazolinium-methosulfat,
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin und Bisabolol,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Quell- und Penetrationsstoffe wie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann, bekannten einschlägigen Handbücher, z. B. die oben genannte Monographie von K. H. Schrader sowie verwiesen.

Die erfindungsgemäßen Zubereitungen können Einsatz in Mitteln zur Haarpflege wie Shampoos, Konditioniermitteln, Spülungen, Aerosolen und Gelen sowie in Haarfärbemitteln finden oder auch in Mitteln zur Textil- oder Faserbehandlung in Form von Waschmitteln, Weichspülern, Imprägnierungen und Appreturen verwendet werden.

Die folgenden Beispiele sollen die Erfindung näher erläutern.

### Beispiele

Soweit nicht anders angegeben, sind alle Prozentangaben als Gew.-% zu verstehen, und alle Mengenangaben als Gewichtsteile.

Zur Verdeutlichung der erfindungsgemäßen Effekte wurde eine Kombination aus Bernsteinsäure und Cystin während eines Kaltwellprozesses eingearbeitet (Haartyp: Natural dark brown code #6634 der Firma Alkinco). Dabei blieben die Einwirkzeiten im Dauerwellprozess unverändert. Die Behandlungstemperatur lag bei 32°C.

### 1. Messapparaturen

Zum Nachweis der erfindungsgemäßen Effekte wurden mit Hilfe eines Zug-Dehnungsgerätes der Firma Dia-Stron (MTT 670) Spannungswerte, Gradienten, Elastizitäts-Modul, Reißdehnung und Reißspannung der nassen Haare bestimmt. Die Bestimmung der Haarquerschnittsfläche der nassen Einzelhaare erfolgte mittels berührungsloser Projektionsmessung durch im Stand der Technik bekannte Lasertechnik. Dazu wurde ein Universal-Dimensionsmesser vom Typ UMD5000A der Firma Zimmer verwendet.

### 2. Statistische Auswertung

Durch den T-Test, eine statistische Auswertung, mit der die Messreihen zweiseitig, paarweise verglichen werden, erhält man prozentuale Wahrscheinlichkeiten, mit der die Messreihen unterschieden sind (Unterscheidung: 90-95 % Messreihen sind tendenziell unterschieden, >95% Messreihen signifikant unterschieden, >99% Messreihen hochsignifikant unterschieden).

### 3. Restrukturierungsuntersuchungen

### 3.1 Haarbehandlung

Es wurden 40 Einzelhaare in zwei Teile geteilt. Der eine Teil wurde durch zwei Kaltwellen geschädigt, der andere Teil wurde mit zwei Kaltwellen behandelt, bei denen zum Reduktionsmittel zuvor eine erfindungsgemäße Kombination aus Bernsteinsäure und Cystin bzw. zum Vergleich nur einer dieser Stoffe zugegeben wurde. Alle 80 Einzelhaare wurden vor der Ermittlung der Reißkurven einer Haarquerschnittsflächenbestimmung im nassen Zustand unterzogen.

### 3.2 Behandlungsschritte:

In den nachfolgenden Experimenten kamen folgende Behandlungsschritte zum Einsatz.
a) 30 Min Applikation einer Kaltwelle (7% TGA = Thioglykolsäure, 0,3% Turpinal SL = 1-Hydroxyethan-1,1-diphosphonsäure, 3,5% (NH₄)₂CO₃, pH-Wert 8,4). Anschließend wurden die Haare 5 Minuten mit Wasser gespült.
b) 30 Min Applikation einer Kaltwelle (17% Ammoniumthioglykolat (71%ig), 0,3% Turpinal SL, 2,5% Ammoniak (25%ig), 5% Ammoniumhydrogencarbonat, 1 % Cremophor RH 40 (hydriertes Ricinusöl + 40-45 Ethylenoxid; INCl-Bezeichnung: PEG-40 Hydrogenated Castor Oil, Fa. BASF), 1 % Lamepon S (Protein-Kokosfettsäurekondensat, Kaliumsalz; INCI-Bezeichnung: Potassium Cocoyl Hydrolyzed Collagen, Aktivstoffgehalt ca. 32%, Fa. COGNIS), 0,5% Parfüm, 0,1 % Gluadin WQ (Laurdimonium hydroxypropyl hydrolyzed wheat protein)" 0,1% Merquat 100 (Polydimethyldiallylammoniumchlorid), Wasser ad 100). Anschließend werden die Haare 5 Minuten mit Wasser ausgespült.
c) 10 Min Applikation der Fixierung (2% H₂O₂, 1% Turpinal SL, pH-Wert 4,0). Anschließend wurden die Haare 5 Minuten mit Wasser gespült.
d) 10 Min Applikation der Fixierung (5% H₂O₂, 0,2% NH₃, 1,7% Turpinal SL, 6% Texapon NSO UP (Natriumlaurylethersulfat), Wasser vollentsalzt ad 100%). Anschließend werden die Haare 5 Minuten mit Wasser ausgespült.
e) Mindestens 24h Lagerung der Haare bei RT (ca. 20°C)
f) Vermessung der Haarquerschnitte der nassen Einzelhaare.
g) Bestimmung der Reißwerte der nassen Einzelhaare.

### 3.3 Ergebnisse Kaltwelle mit 1 % Cystin

Referenzbeispiel: zweifach dauergewellt, wie unter 3.2 beschrieben: Schritte a), c) e); Wiederholung der Schritte a), c), e); dann f) und g).
Vergleichsbeispiel: analoge Behandlung, wobei der in Schritt a) verwendeten Kaltwelle 1 % L-Cystin zugesetzt wurde.

| | | | | |
|---|---|---|---|---|
| Elastic E-Modul | Elastic Gradient | Spannung Plateaubereich | Spannung bei 15% Dehnung | Arbeit bei 15% Dehnung |
| [N/m²] | [N/mm] | [N/µm²] | [N/Nm²] | [J] |
| Referenzbeispiel | | | | |
| 7.07E+08 | 1,08E-01 | 2,46E-05 | 2,33E-05 | 4,26E-04 |
| Vergleichsbeispiel mit 1 % Cystin | | | | |
| 7,81 E+08 | 1,22E-01 | 2,62E-05 | 2,54E-05 | 4,80E-04 |
| T-Test paarweise, zweiseitig | | | | |
| signifikant unterschieden | signifikant unterschieden | signifikant unterschieden | signifikant unterschieden | signifikant unterschieden |
| | | | | |
| Spannung bei 25% Dehnung | Arbeit bei 25% Dehnung | Reiß-Dehnung | | |
| [N/µm²] | [J] | [%] | | |
| Referenzbeispiel | | | | |
| 2.88E-05 | 8,08E-04 | 5.87E+01 | | |
| Vergleichsbeispiel mit 1 % Cystin | | | | |
| 3,15E-05 | 9,10E-04 | 5,45E+01 | | |
| T-Test paarweise, zweiseitig | | | | |
| signifikant unterschieden | signifikant unterschieden | signifikant unterschieden | | |

Ergebnis: Die Spannungs- und Arbeitswerte des plastischen Bereiches, sowie das E-Modul und der Gradient sind signifikant unterschieden. Durch die Behandlung mit Dauerwelle und einem Zusatz von 1 % L-Cystin war eine signifikante Haarverstärkung festzustellen, d.h. durch Zusatz von Cystin zum Wellmittel konnte der durch die Dauerwelle verursachte Haarschaden reduziert werden.

### 3.4 Ergebnisse Kaltwelle mit 1 % Cystin-Bernsteinsäure-Gemisch (1:1)

Referenzbeispiel: zweifach dauergewellt, wie unter 3.2 beschrieben: Schritte a), c) e); Wiederholung der Schritte a), c), e); dann f) und g).
Erfindungsgemäßes Beispiel: analoge Behandlung, wobei der in Schritt a) verwendeten Kaltwelle 1 % eines Gemischs aus gleichen Gewichtsteilen L-Cystin und Bernsteinsäure zugesetzt worden waren.

| | | | | |
|---|---|---|---|---|
| Elastic E-Modul | Elastic Gradient | Spannung Plateaubereich | Spannung bei 15% Dehnung | Arbeit bei 15% Dehnung |
| [N/m²] | [N/mm] | [N/µm²] | [N/µm²] | [J] |
| Referenzbeispiel | | | | |
| 8,57E+08 | 1,25E-01 | 3,04E-05 | 2,85E-05 | 4,85E-04 |
| Erfindungsgemäßes Beispiel mit 1 % Bernsteinsäure/L-Cystin-Gemisch | | | | |
| 9,30E+08 | 1,43E-01 | 3,19E-05 | 3,07E-05 | 5,52E-04 |
| T-Test paarweise, zweiseitig | | | | |
| signifikant unterschieden | signifikant unterschieden | signifikant unterschieden | signifikant unterschieden | signifikant unterschieden |
| | | | | |
| Spannung bei 25% Dehnung | Arbeit bei 25% Dehnung | Reiß-Dehnung | | |
| [N/µm²] | [J] | [%] | | |
| Referenzbeispiel | | | | |
| 3,52E-05 | 9,20E-04 | 5,98E+01 | | |
| Erfindungsgemäßes Beispiel mit 1 % Bernsteinsäure/L-Cystin-Gemisch | | | | |
| 3,74E-05 | 1,04E-03 | 5,59E+01 | | |
| T-Test paarweise, zweiseitig | | | | |
| signifikant unterschieden | signifikant unterschieden | signifikant unterschieden | | |

Ergebnis: Durch die Behandlung mit der Dauerwelle und dem Zusatz an 1 % Bernsteinsäure/L-Cystin -Gemisch war eine signifikante Haarstärkung zu sehen. Das E-Modul und der Gradient, sowie und Spannungs- und Arbeitswerte des plastischen Bereichs sind signifikant größer als bei der Referenzdauerwelle. Die Reißdehnung ist im Vergleich zur Referenz signifikant kleiner, was ebenfalls eine Restrukturierung anzeigt.
Diese Strukturverbesserung ist größer als die durch Cystin alleine gemessene (vgl. Versuchsergebnisse unter 3.3). Dies zeigt der Vergleich der Versuchsergebnisse unter 3.3 mit denen unter 3.4 mit Hilfe der Covarianzanalyse auf Basis multipler Vergleiche nach Scheffée:

### Statistische Auswertung:

Zum Nachweis der Wirkung des Bernsteinsäure/L-Cystin-Gemisches im Vergleich zum L-Cystin wurden die beiden Behandlungsgruppen unter Berücksichtigung ihrer Anfangswerte (nur kaltgewellt) mit der Covarianzanalyse auf Basis multipler Vergleiche nach Scheffèe verglichen.

Es wurden folgende Signifikanzen festgestellt:

| | | | | |
|---|---|---|---|---|
| Elastic E-Modul | Elastic Gradient | Spannung Plateaubereich | Spannung bei 15% Dehnung | Arbeit bei 15% Dehnung |
| | | | | |
| tendeziell unterschieden | tendeziell unterschieden | Signifikant unterschieden | signifikant unterschieden | tendeziell unterschieden |
| | | | | |
| Spannung bei 25% Dehnung | Arbeit. bei 25% Dehnung | Reiß-Dehnung | | |
| [N/µm²] | [J] | [%] | | |
| | | | | |
| tendeziell unterschieden | nicht unterschieden | nicht unterschieden | | |

Gesamtergebnis: Die restrukturierende Wirkung des L-Cystins wurde bereits festgestellt. Bei der Applikation von 1 % Bernsteinsäure/L-Cystin (1:1) in der Dauerwelle ist ebenfalls eine signifikante Restrukturierung nachzuweisen. Mittels Covarianzanalyse konnte eine signifikant stärkere Restrukturierung der Haare durch Zugabe eines Bernsteinsäure/L-Cystin -Gemisches in die Dauerwelle im Vergleich zu L-Cystin nachgewiesen werden, da E-Modul und Gradient, sowie Spannungs- und Arbeitswerte des plastischen Bereichs unterschieden sind. Somit kann von einem synergistischern Effekt durch die Kombination von Cystin und Bernsteinsäure gesprochen werden.

### 3.5. Ergebnisse in einer handelsüblichen Kaltwelle mit 1% Bernsteinsäure:

Referenzbeispiel: zweifach mit einer handelsüblichen Dauerwelle dauergewellt: wie unter 3.2 beschrieben Schritte b), d) e); Wiederholung der Schritte b), d), e); dann f) und g).
Vergleichsbeispiel: analoge Behandlung wie Referenzbeispiel, wobei der in Schritt b) verwendeten Kaltwelle 1 % Bernsteinsäure zugesetzt wurde

| | | | | |
|---|---|---|---|---|
| Elastic E-Modul | Elastic Gradient | Spannung Plateaubereich | Spannung bei 15% Dehnung | Arbeit bei 15% Dehnung |
| [N/m²] | [N/mm] | [N/µm²] | [N/µm²] | [J] |
| Referenz zweifach durch KW geschädigt | | | | |
| 8,09E+08 | 1,18E-01 | 2,83E-05 | 2,72E-05 | 4,65E-04 |
| Vergleichsbeispiel zweifach kaltgewellt mit Zusatz an 1% Bernsteinsäure | | | | |
| 8,76E+08 | 1,26E-01 | 3,08E-05 | 2,91 E-05 | 5,02E-04 |
| t-Test, zweiseitig, paarweise | | | | |
| signifikant unterschieden | signifikant unterschieden | signifikant unterschieden | signifikant unterschieden | signifikant unterschieden |
| | | | | |
| Spannung bei 25% | Arbeit bei 25% | Reiß-Spannung | Gesamtarbeit | |
| Dehnung | Dehnung | | | |
| [N/µm²] | [J] | [N/µm²] | [J] | |
| Referenz zweifach durch KW geschädigt | | | | |
| 3,49E-05 | 8,94E-04 | 1,22E-04 | 4,14E-03 | |
| Vergleichsbeispiel zweifach kaltgewellt mit Zusatz an 1 % Bernsteinsäure | | | | |
| 3,69E-05 | 9,58E-04 | 1,34E-04 | 4,60E-03 | |
| t-Test, zweiseitig, paarweise | | | | |
| signifikant unterschieden | signifikant unter schieden | signifikant unterschieden | signifikant unterschieden | |

Ergebnis: Bernsteinsäure führt zu einer signifikanten Haarverstärkung. Durch Zugabe von Bernsteinsäure im Dauerwellprozess kann man eine Erhöhung des E-Moduls, des Gradienten, der Spannungswerte, der Arbeit beobachten, woraus man eine Verbesserung der Struktur ablesen kann.

### 3.6. Ergebnisse in einer handelsüblichen Kaltwelle mit 1 % Bemsteinsäure/Cystin-Gemisch:

Referenzbeispiel: zweifach mit einer handelsüblichen Dauerwelle dauergewellt: wie unter 3.2 beschrieben Schritte b), d) e); Wiederholung der Schritte b), d), e); dann f) und g).
Erfindungsgemäßes Beispiel: analoge Behandlung wie Referenzbeispiel, wobei der in Schritt b) verwendeten Kaltwelle 1 % Bernsteinsäure/L-Cystin-Gemisch 1:1 (gleiche Gewichtsteile) zugesetzt wurde

| | | | | |
|---|---|---|---|---|
| Elastic E-Modul | Elastic Gradient | Spannung Plateaubereich | Spannung bei 15% Dehnung | Arbeit bei 15% Dehnung. |
| [N/m²] | [N/mm] | [N/µm²] | [N/µm²] | [J] |
| Referenz zweifach durch KW geschädigt | | | | |
| 7,16E+08 | 1,02E-01 | 2,61 E-05 | 2,46E-05 | 3,87E-04 |
| Erfindungsgemäßes Beispiel mit 1 % Bernsteinsäure/L-Cystin-Gemisch | | | | |
| 8,47E+08 | 1,18E-01 | 2,90E-05 | 2,75E-05 | 4,39E-04 |
| t-Test, zweiseitig, paarweise | | | | |
| signifikant unterschieden | signifikant unterschieden | signifikant unterschieden | signifikant unterschieden | signifikant unterschieden |
| | | | | |
| Spannung bei 25% Dehnung | Arbeit bei 25% Dehnung | | | |
| [N/µm²] | [J] | | | |
| Referenz zweifach durch KW geschädigt | | | | |
| 3,11E-05 | 7,42E-04 | | | |
| Erfindungsgemäßes Beispiel mit 1 % Bernsteinsäure/L-Cystin-Gemisch | | | | |
| 3,39E-05 | 8,31 E-04 | | | |
| t-Test, zweiseitig, paarweise | | | | |
| signifikant unter schieden | signifikant unterschieden | | | |

Ergebnis: Durch die erfindungsgemäße Behandlung war eine signifikante Haarverstärkung festzustellen. Die Spannungs- und Arbeitswerte des plastischen Bereiches, sowie das E-Modul und der Gradient sind signifikant unterschieden. Diese Haarverstärkung ist auch deutlich größer als die durch Bernsteinsäure alleine bewirkte (vgl. Beispiel 3.5). Es liegt demnach bei dem Gemisch aus Bernsteinsäure und Cystin ein synergistischer Effekt vor.

## Patentansprüche

1. Verfahren zur Restrukturierung keratinischer Fasern, bei dem eine Keratinfaser mit Cystin und mit mindestens einer Dicarbonsäure mit 2 bis 10 Kohlenstoffatomen in Kontakt gebracht wird.

2. Verfahren nach Anspruch 1, **dadurch gekennzeichnet, daß** es sich bei der Keratinfaser um ein Haar handelt.

3. Verfahren nach Anspruch 1 oder 2, **dadurch gekennzeichnet, daß** die Restrukturierung eine Faserverstärkung umfaßt.

4. Verfahren nach einem der Ansprüche 1 bis 3, **dadurch gekennzeichnet, daß** die Dicarbonsäure ausgewählt ist aus der Gruppe, die gebildet wird von Dicarbonsäuren HOOC-(CH₂)ₙ-COOH mit n=0 bis 8, Maleinsäure, Fumarsäure, Sorbinsäure sowie Gemischen dieser Stoffe.

5. Verfahren nach Anspruch 4, **dadurch gekennzeichnet, daß** es sich bei der Dicarbonsäure um Bernsteinsäure handelt.

6. Verfahren nach einem der Ansprüche 1 bis 5, **dadurch gekennzeichnet, daß** Cystin und die mindestens eine Dicarbonsäure in einem Gewichtsverhältnis von 99 zu 1 bis 1 zu 99 vorliegen.

7. Verfahren nach Anspruch 6, **dadurch gekennzeichnet, daß** Cystin und die mindestens eine Dicarbonsäure in einem Gewichtsverhältnis von 10 zu 1 bis 1 zu 10, insbesondere aber von 2 zu 1 bis 1 zu 2 vorliegen.

8. Zubereitung zur Verwendung in einem Verfahren nach einem der Ansprüche 1 bis 7, umfassend
(a) 0,01 bis 20 Gew.-% Cystin
(b) 0,01 bis 20 Gew.-% mindestens einer Dicarbonsäure mit 2 bis 10 Kohlenstoffatomen

9. Zubereitung nach Anspruch 8, **dadurch gekennzeichnet, daß** die Dicarbonsäure ausgewählt ist aus der Gruppe, die gebildet wird von Dicarbonsäuren HOOC-(CH₂)ₙ-COOH mit n=0 bis 8, Maleinsäure, Fumarsäure, Sorbinsäure sowie Gemischen dieser Stoffe, und insbesondere Bernsteinsäure bedeutet.

10. Verwendung einer Kombination aus Cystin und mindestens einer Dicarbonsäure mit 2 bis 10 Kohlenstoffatomen zur Restrukturierung von keratinischen Fasern, insbesondere von Haaren.

11. Verwendung nach Anspruch 10, **dadurch gekennzeichnet, daß** die Restrukturierung eine Faserverstärkung umfaßt.

## Claims

1. A method for restructuring keratinic fibers, in which method a keratin fiber is brought into contact with cystine and with at least one dicarboxylic acid having 2 to 10 carbon atoms.

2. The method according to Claim 1, wherein the keratin fiber is a hair.

3. The method according to Claim 1 or 2, wherein the restructuring encompasses a fiber reinforcement.

4. The method according to one of Claims 1 to 3, wherein the dicarboxylic acid is selected from the group constituted by HOOC-(CH₂)ₙ-COOH dicarboxylic acids where n = 0 to 8, maleic acid, fumaric acid, sorbic acid, and mixtures of these substances.

5. The method according to Claim 4, wherein the dicarboxylic acid is succinic acid.

6. The method according to one of Claims 1 to 5, wherein cystine and the at least one dicarboxylic acid are present at a weight ratio from 99 to 1 to 1 to 99.

7. The method according to Claim 6, wherein cystine and the at least one dicarboxylic acid are present at a weight ratio from 10 to 1 to 1 to 10, but in particular from 2 to 1 to 1 to 2.

8. A preparation for use in a method according to one of Claims 1 to 7, encompassing
(a) 0.01 to 20 wt% cystine
(b) 0.01 to 20 wt% of at least one dicarboxylic acid having 2 to 10 carbon atoms.

9. The preparation according to Claim 8, wherein the dicarboxylic acid is selected from the group constituted by HOOC-(CH₂)ₙ-COOH dicarboxylic acids where n = 0 to 8, maleic acid, fumaric acid, sorbic acid, and mixtures of these substances, and in particular denotes succinic acid.

10. A use of a combination of cystine and at least one dicarboxylic acid having 2 to 10 carbon atoms, for the restructuring of keratinic fibers, in particular of hair.

11. The use according to Claim 10, wherein the restructuring encompasses a fiber reinforcement.

## Revendications

1. Procédé pour la restructuration de fibres kératiniques, dans lequel une fibre kératinique est mise en contact avec de la cystine et au moins un acide dicarboxylique comprenant 2 à 10 atomes de carbone.

2. Procédé selon la revendication 1, **caractérisé en ce qu'**il s'agit, pour la fibre kératinique, d'un cheveu.

3. Procédé selon la revendication 1 ou 2, **caractérisé en ce que** la restructuration comprend un renforcement de la fibre.

4. Procédé selon l'une quelconque des revendications 1 à 3, **caractérisé en ce que** l'acide dicarboxylique est choisi dans le groupe formé par les acides dicarboxyliques HOOC-(CH₂)ₙ-COOH avec n=0 à 8, l'acide maléique, l'acide fumarique, l'acide sorbique ainsi que les mélanges de ces substances.

5. Procédé selon la revendication 4, **caractérisé en ce qu'**il s'agit, pour l'acide dicarboxylique, de l'acide succinique.

6. Procédé selon l'une quelconque des revendications 1 à 5, **caractérisé en ce que** la cystine et ledit au moins un acide dicarboxylique se trouvent dans un rapport pondéral de 99:1 à 1:99.

7. Procédé selon la revendication 6, **caractérisé en ce que** la cystine et ledit au moins un acide dicarboxylique se trouvent dans un rapport pondéral de 10:1 à 1:10, en particulier toutefois de 2:1 à 1:2.

8. Composition pour une utilisation dans un procédé selon l'une quelconque des revendications 1 à 7, comprenant
(a) 0,01 à 20% en poids de cystine
(b) 0,01 à 20% en poids d'au moins un acide dicarboxylique comprenant 2 à 10 atomes de carbone.

9. Composition selon la revendication 8, **caractérisée en ce que** l'acide dicarboxylique est choisi dans le groupe formé par les acides dicarboxyliques HOOC-(CH₂)ₙ-COOH avec n=0 à 8, l'acide maléique, l'acide fumarique, l'acide sorbique ainsi que les mélanges de ces substances et signifie en particulier l'acide succinique.

10. Utilisation d'une combinaison de cystine et d'au moins un acide dicarboxylique comprenant 2 à 10 atomes de carbone pour la restructuration de fibres kératiniques, en particulier de cheveux.

11. Composition selon la revendication 10, **caractérisée en ce que** la restructuration comprend un renforcement de la fibre.
